# EUROPEAN PATENT SPECIFICATION

(11) **EP 2 874 687 B1**
(45) Date of publication and mention of the grant of the patent: **06.03.2019**
(21) Application number: 13823262.4
(22) Date of filing: 23.07.2013
(51) Int. Cl.: A61M 16/04, A61M 25/00, A61M 25/06

(54) **DEVICE FOR EMERGENCY APNEIC OXYGENATION**
VORRICHTUNG ZUR SAUERSTOFFZUFÜHRUNG FÜR APNOISCHEN NOTFALL
DISPOSITIF D'OXYGÉNATION APNÉIQUE D'URGENCE

(30) Priority: 23.07.2012 US 201261674414 P
(43) Date of publication of application: 27.05.2015
(73) Proprietor: University of Maryland, Baltimore, Baltimore, MD 21201 (US)
(72) Inventor: WOLF, Jeffrey S., Owings Mills, Maryland 21117 (US); IACONO, Aldo T., Cockeysville, Maryland 21030 (US)
(74) Representative: Chapman, Paul Gilmour
(86) International application number: PCT/US2013/051739
(87) International publication number: WO 2014/018565

(56) References cited:
- EP-A1- 1 393 768
- US-A- 4 677 978
- US-A- 5 251 616
- US-A- 5 967 143
- US-A1- 2006 015 131
- US-A1- 2009 229 614
- US-A1- 2009 260 625
- US-A1- 2011 041 854
- US-A1- 2012 145 147
- US-B2- 7 341 061

## Description

### RELATED APPLICATIONS

This application claims benefit of Provisional Appln. 61/674,414, filed July 23, 2012.

### BACKGROUND OF THE INVENTION

Apnea refers to suspension of external breathing. During apnea there is little or no movement of the muscles of respiration and the volume of the lungs essentially remains unchanged. Severe tissue damage, brain damage and death can result. Oxygenation during apnea is called apneic oxygenation. Continuous apneic oxygenation delivered to the lower end of the trachea has been found to maintain trauma patients for up to one hour following injury. Despite these findings, there has yet to be an apneic oxygenation catheter developed for use in the field by emergency medical technicians (EMTs) or the military.

A cricothyrotomy is an incision through the cricothyroid membrane above the cricoid cartilage readily evident just above the trachea, and is considered less invasive than an incision through the trachea (tracheotomy) and to have fewer complications. Cricothyrotomy ventilation is often necessary to secure the airway in injuries requiring apneic oxygenation. When there is an obstruction in the airway and endotracheal intubation is not possible, an immediate solution is to insert a tube through a hole in the cricothyroid membrane. In some cases the bypass will allow the patient to breathe on their own. In other instances the bypass will provide an entry way for assisted ventilation and/or drug delivery.

Generally, the devices available to perform emergency cricothyrotomies require a skilled practitioner and require many steps to secure the airway. One example device and procedure are described in United States Patent number 4,677,978. There, a derivative of the Seldinger method is used making the installation of this device labor intensive. First, a scalpel is used to make an incision into the cricothyroid membrane. Next, an over-the-needle catheter is entered into the airway with a syringe. The syringe and needle are then removed, leaving the catheter in place. Following that, a guide wire is inserted into the catheter, and the catheter is removed. Finally a dilator is inserted over the guide wire and the guide wire is removed.

Other devices such as those described in United States Patent number 4,869,718 do not use the Seldinger method and therefore require fewer steps. However, these devices only provide a small opening for the catheter and are limited to high frequency jet ventilation.

US5967143 discloses a surgical instrument, especially for emergency medicine, in particular a so-called coniotomy device with which access to the lung is created for supplying air to the lung in cases in which breathing is hindered in the larynx, which device can be fixated internally and externally and has a short, straight indwelling cannula with a fixative rim at its end and with a trocar, the cutting edge of which is perpendicular to the cricoid cartilage, does not allow any wall contacts within the trachea, causes no injuries to the elastic fibers of the ligamentum conicum and can also be equipped with a small closure sac inserted into the trachea along with the cannula, which can be blown up from outside and which makes it possible to close the trachea in the upwards direction, preventing any escape from the trachea of the breathing air blown into the indwelling cannula.

EP1393768 A1 discloses an instrument for providing ventilation access to the trachea having a hollow needle with a sharp tip, a tube extending within the needle and a spring urging the tube to project a short distance from the patient end of the needle. The rear end of the tube carries two coloured flags one of which is visible at a time in a transparent window towards the machine end of the needle. When the patient end of the instrument is pushed through neck tissue overlying the trachea the inner tube is pushed rearwardly so that a flag of one colour is visible. When the patient end of the instrument enters the trachea the spring can push the inner tube forwardly so that the other flag is visible. In one embodiment the instrument carries a dilator and a tracheostomy tube that can be slid off the needle after penetration. In another embodiment, ventilation equipment is connected to the rear of the inner tube and the patient is ventilated through the instrument itself.

### SUMMARY OF THE INVENTION

According to the invention there is provided a cannula for emergency apneic oxygenation comprising: a longitudinal inner passage having an inner diameter; a distal portion having a first outer diameter greater than the inner diameter, wherein the cannula is shaped in the distal portion to bend in a first direction along the inner passage, and wherein the distal portion is made of shape memory material; and a cannula base), wherein the cannula base comprises a distal face having a second outer diameter greater than the first outer diameter, and wherein a distance from a distal end of the cannula to a proximal end of the distal portion of the cannula is less than a distance from a surface of a throat of a target subject to a distal surface of an airway of the target subject, and the inner passage is configured to pass a catheter connected at a proximal end to an oxygen source.

The invention further provides a system and a kit for emergency apneic oxygenation comprisng a cannula according to the invention.

Techniques are provided for emergency apneic oxygenation, including devices that provide a more sustainable opening through the cricothyroid membrane.

In a first set of examples, a cannula for emergency apneic oxygenation includes a longitudinal inner passage having an inner diameter. A distal portion of the cannula is made of shape memory material shaped to bend in a first direction along the inner passage, and has a first outer diameter greater than the inner diameter. The cannula includes a cannula base having a second outer diameter greater than the first outer diameter. A distance from a distal end of the cannula to a proximal end of the distal portion of the cannula is less than a distance from a surface of a throat of a target subject to a distal surface of an airway of the target subject.

In some of examples of the first set, the first outer diameter is less than 10 millimeters.

In a second set of examples, a catheter for emergency apneic oxygenation includes a distal portion having a first outer diameter and a first longitudinal inner passage of a first inner diameter less than the first outer diameter. The catheter also includes a proximal portion configured at a proximal end for attachment to a fluid supply and having a second longitudinal inner passage in fluid communication with the first longitudinal inner passage. The catheter still further includes padding at the distal end of the distal portion configured to disperse fluid flow and to prevent damage to a lining of an airway of a target subject.

In some examples of the second set, the first outer diameter is less than 10 millimeters.

In some examples of the second set, the catheter includes a mark or a collar configured to be placed around the catheter at a particular distance to the proximal side from the distal end of the distal portion. The particular distance is approximately equal to a distance from an entry point into the airway of the target subject to a sub-segmented bronchus of the target subject. In some of these embodiments, the particular distance is in a range from about 5 centimeters to about 15 centimeters.

In a third set of examples, a trocar for emergency apneic oxygenation includes a distal portion comprising a tapered cutting edge and a penetration portion disposed proximal to the distal portion and having a diameter less than 10 millimeters. The trocar also includes a stop lip disposed proximal to the penetration portion and having a diameter greater than the diameter of the penetration portion. A distance from a distal end of the stop lip to a distal end of the distal portion is less than about a distance from a surface of a throat of a target subject to a distal surface of an airway of the target subject.

In a fourth set of examples, a system for emergency apneic oxygenation includes a cannula and a trocar. The cannula includes an inner passage of an inner diameter, a distal portion and a cannula base. The distal portion has a first outer diameter greater than the inner diameter, and is made of shape memory material shaped to bend in a first direction along the inner passage. The cannula base has a second outer diameter greater than the first outer diameter. The trocar includes a distal portion that includes a cutting edge, a penetration portion and a stop lip. The penetration portion is disposed proximal to the distal portion and has a diameter about equal to the inner diameter. The stop lip is disposed proximal to the penetration portion and has a diameter greater than the diameter of the penetration portion. The trocar is configured to engage the cannula by passing through the inner passage and straightening the bent distal portion of the cannula. When the trocar is engaged, a distance from a distal end of the distal portion of the trocar to a proximal end of the distal portion of the cannula is less than a distance from a surface of a throat of a target subject to a distal surface of an airway of the target subject.

In some examples of the fourth set, the system also includes a system base that has a system base opening that has a diameter about equal to the first outer diameter. The system base has an area outside the system base opening that is sufficient to inhibit the cannula base from passing into the airway of the target subject.

In some examples of the fourth set, the system also includes a catheter. The catheter is configured to pass through the inner passage of the cannula and be directed by the direction of the bent distal portion of the cannula down the airway of the target subject, after the cannula passes into the airway of the target subject and the trocar is removed.

In a fifth set of examples, a kit for emergency apneic oxygenation includes a cannula, a trocar, a base and a catheter. The cannula includes an inner passage of an inner diameter, a distal portion, and a cannula base. The distal portion has a first outer diameter greater than the inner diameter, and is made of shape memory material shaped to bend in a first direction along the inner passage. The cannula base has a second outer diameter greater than the first outer diameter. The trocar is configured to engage the cannula by passing through the inner passage and straightening the bent distal portion of the cannula. The system base has an opening about equal to the first outer diameter and is configured to be placed with the opening centered on an appropriate entry site on a target subject for the trocar engaged with the cannula. The catheter is configured to pass through the cannula after insertion of the cannula into the entry site by the engaged trocar and subsequent removal of the trocar. The catheter has a length that is at least a sum of a first distance from the entry site to a sub-segmented bronchus of the target subject and a second distance from the entry site to a supply of fluid.

In some examples of the fifth set, the first outer diameter is less than 10 millimeters.

Also disclosed is a method for emergency apneic oxygenation including cutting an opening of diameter less than 10 millimeters into an airway of a target subject at an entry site. The method also includes passing a distal end of a catheter through the opening and down the airway of the target subject to a sub-segmented bronchus of the target subject. The method further includes connecting a distal end of the catheter to a supply of oxygen and providing oxygen from the supply to the target subject at a rate sufficient to sustain life of the target subject.

Still other aspects, features, and advantages of the invention are readily apparent from the following detailed description, simply by illustrating a number of particular embodiments and implementations, including the best mode contemplated for carrying out the invention. The invention is also capable of other and different embodiments, and its several details can be modified in various obvious respects, all without departing from the scope of the invention. Accordingly, the drawings and description are to be regarded as illustrative in nature, and not as restrictive.

### BRIEF DESCRIPTION OF THE DRAWINGS

The present invention is illustrated by way of example, and not by way of limitation, in the figures of the accompanying drawings and in which like reference numerals refer to similar elements and in which:
FIG. 1A through FIG.1D are block diagrams that illustrate example components of a apnea oxygenation kit, according to an embodiment;
FIG. 2A through FIG. 2D are block diagrams that illustrate example use of the components of FIG. 1A through FIG. 1D, according to an embodiment;
FIG. 3A through FIG. 3E and FIG. 4 are block diagrams that illustrate example variations to the kit of FIG. 1A through FIG. 1D, including a protective casing according to various embodiments;
FIG. 4A and FIG. 4B are block diagrams that illustrate a trocar with adjustable diameter cutting edge, according to an embodiment; and
FIG. 5A through FIG 5H are block diagrams that illustrate example variations in catheters from that depicted in FIG. 1D, according to various embodiments.

### DETAILED DESCRIPTION

A method, apparatus, system and kit are described for emergency apneic oxygenation. In the following description, for the purposes of explanation, numerous specific details are set forth in order to provide a thorough understanding of the present invention. It will be apparent, however, to one skilled in the art that the present invention may be practiced without these specific details. In other instances, well-known structures and devices are shown in block diagram form in order to avoid unnecessarily obscuring the present invention.

Some embodiments of the invention are described below in the context of an emergency, such a trauma caused by natural disasters, accidents, or acts of war or terror, suffered by an adult. However, the invention is not limited to this context. In other embodiments the procedure or device is employed on children and in clinical or hospital settings, such as in first aid, preparation for or recovery from surgery, or response to power failures in the operating room, or wherever cardiopulmonary resuscitation (CPR) or automated defribrillator is employed, such as for response to heart attack, pulmonary embolism, significant overwhelming infection, and choking.

As used herein, a "proximal" end or face shall be construed as the end or face that is closest to the user when the device is in use. As defined herein, a "distal" end or face shall be understood as the end or face that is closest to, or deepest inside, the patient, and farthest from the user, when the device is in use. As used herein, diameter refers to a shortest distance through an object, whether the object has a circular cross section or not. As used herein, a subject is a person or animal, and a target subject is a subject that is to receive apneic oxygenation. In some embodiments, the target subject is an individual person; in some embodiments, the target subject is a population of individuals, such as adults or sub-teenaged children. In such embodiments, the values of characteristics (such as values of airway diameter and length) of the target subject are an average or range of characteristics of the population. As used herein, a fluid means any material that flows at ambient temperatures, including liquids (e.g., medications) and gases (e.g., oxygen gas).

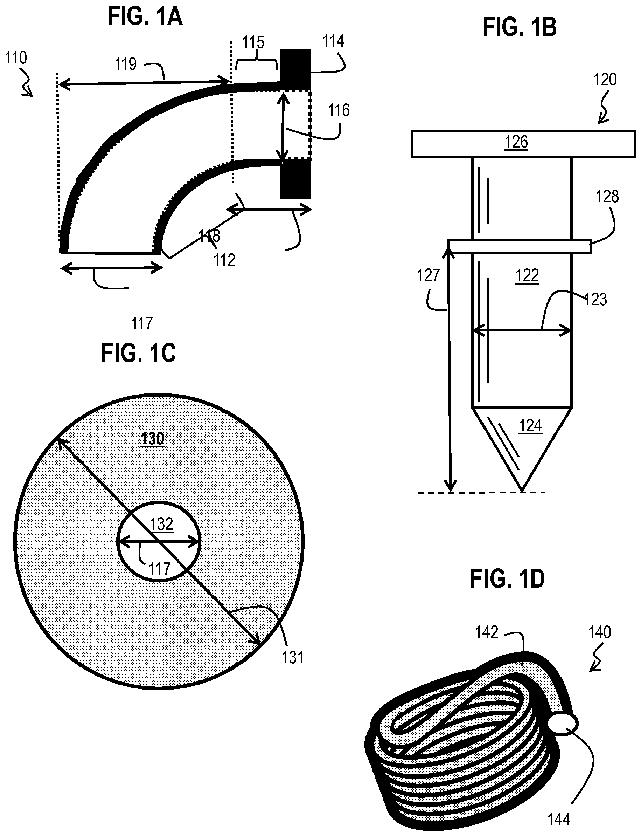

FIG. 1A through FIG.1D are block diagrams that illustrate example components of an apnea oxygenation kit, according to an embodiment. In the illustrated embodiment, the components of the kit are shown with circular cross sections; however, in other embodiments other cross sections are used, such as oval cross sections, polygonal cross sections, lens shaped cross sections, and rectilinear cross sections.

FIG. 1A is a block diagram that illustrates an example cross section through a cannula 110 with an inner passage of inner diameter 116 configured for passing one or more catheters. The cannula 110 is configured to be inserted through a wall at the front of the target subject's airway, e.g., above the cricoid cartilage, and into the target subject's airway. Thus it extends from the skin surface of a throat of a target subject into the airway.

A distal portion 112 of the cannula is made of a shape memory material and is bent in a first direction (downward in the illustrated view) as one progresses through the inner passage from a proximal end to a distal end. The distal portion 112 has an outer diameter 117, larger than the inner diameter 116. At the proximal end of cannula 110 is a cannula base 114, with an outer diameter greater than the outer diameter 117 of the distal portion 112 of the cannula. In some embodiments, there is a straight portion 115 of the cannula between the bent distal portion 112 and the cannula base portion 112. Suitable shape memory materials are known in the art, for example, titanium, thin stainless steel, and nickel titanium alloy (also called Nitinol). When in place in the wall of a target subject's airway, the downward bend of the distal portion 112 of the cannula directs a catheter threaded through the cannula downward in the subject's airways toward the lungs. This downward bias provides a very advantageous control when an operator is working in unguided and difficult conditions, such as darkness. The larger outer diameter of the cannula base 114 prevents the cannula 110 from falling through a hole with a diameter closely matching the outer diameter 117 of the distal portion 112 of the cannula, while allowing the entire distal portion 112, and in some embodiments, a straight portion 115 to pass into the hole. A the cannula base 114 has thickness 118 and is made of any suitable rigid or semi-rigid material including the same materials as the distal portion or separate materials such as stainless steel, titanium, nitinol, plastics or other types of polymers, or some combination.

Thus, FIG. 1A depicts a cannula that includes an inner passage of an inner diameter 116, a distal portion 112 and a cannula base 114. The distal portion 112 has a first outer diameter greater 117 than the inner diameter 116. The cannula 110 is shaped in the distal portion 112 to bend in a first (downward) direction along the inner passage. The distal portion 112 is made of shape memory material. The cannula base 114 has a second outer diameter greater than the first outer diameter 117. In some embodiments, the cannula includes a straight portion 115 between the cannula base 114 and the distal portion 112, with an outer diameter about equal to the first outer diameter 117.

It is also desirable that the bend in the cannula take place within the airway of the target subject without contacting or penetrating the back wall of the airway. Thus, it is advantageous for a distance 119 from a distal end of the cannula to a proximal end of the distal portion 112 of the cannula to be less than a distance from a surface of a throat of a target subject to a distal surface of an airway of the target subject. In some embodiments, the length of the distal portion 112 is so constrained. In some embodiments in which the distal face of the cannula base 114 is flush with the skin of the target subject, the distance from the distal face of cannula base 114 to the distal end of the cannula is advantageously less than the distance from a surface of a throat of a target subject to a distal surface of an airway of the target subject to avoid damaging or perforating the back wall of the airway.

Currently, apneic oxygenation uses holes into the airway which are a centimeter (10 millimeters) or more. Preferably, smaller incisions are made to reduce blood loss and chances for complications such as infection. By passing catheters attached to oxygen supply, smaller opening can be used. Thus, in various embodiments, the outer diameter 117 of the distal portion of the cannula is less than 10 millimeters and preferable in a range from about 3 millimeters to about 4 millimeters. The inner diameter 116 is sufficient to pass at least one catheter to supply oxygen and is preferably in a range from about 2 millimeters to about 3 millimeters. Larger inner diameters are used for bigger catheters or for multiple catheters, in various embodiments.

FIG. 1B is a block diagram that illustrates an example trocar 120, according to an embodiment. Trocar 120 (also called an obturator) is used to cut a hole from the skin of the throat into the airway of the target subject without damaging the back wall of the airway of the target subject. The trocar is made of any suitable rigid material, such as stainless steel, carbon steel, titanium, cobalt chrome, plastics or other types of polymers, or some combination. The trocar 120 is further configured to leave the cannula in place in the hole so cut. In the illustrated embodiment, the trocar incudes a tapered piercing tip 124 at a distal end and a penetration portion 122 disposed proximal to the distal portion 124 and having a diameter 123 about equal to an inner diameter 116 of the cannula 110. Thus the diameter 123 is less than about 10 millimeters and preferably less than 4 millimeters, and most preferably for use with a single catheter in a range from about 2 millimeters to about 3 millimeters. Piercing tip 124 is generally conical in shape. However, in some embodiments the piercing tip has more than one cutting edge. The trocar 120 also includes a stop lip 128 disposed proximal to the penetration portion and having a diameter greater than the diameter of the penetration portion. The stop lip 128 is configured to keep a cannula 110 from sliding along the trocar during insertion. In some embodiments, proximal to the stop lip is a handle 126 that is more easily grasped by an operator.

To keep from damaging a back wall of the airway of the target subject, a distance 127 from a distal end of the stop lip 128 to a distal end of the distal portion is less than a distance from a surface of a throat of a target subject to a distal surface of an airway of the target subject. In some embodiments, in which the cannula 110 with base of thickness 118 is disposed distal to the stop lip, a distance 127 minus distance 118 is constrained to be less than the distance from the skin of the throat to the back wall of the airway. In some embodiments, in which the cannula 110 with base of thickness 118 and system base of thickness *t_{sb}* is disposed distal to the stop lip and cannula base 114, a distance 127 minus distance 118 and minus *t_{sb}* is constrained to be less than the distance from the skin of the throat to the back wall of the airway. In various embodiments, depending on the target patient, the distance 127 is selected in a range from about 5 millimeters to about 35 millimeters, and preferably about 25 millimeters. The trocar 120 is configured to engage the cannula 110 by passing the distal end 124 of the trocar through the inner passage of the cannula base 114 and thence into the inner passage of the bent distal portion 112, and straightening the bent distal portion 112 of the cannula.

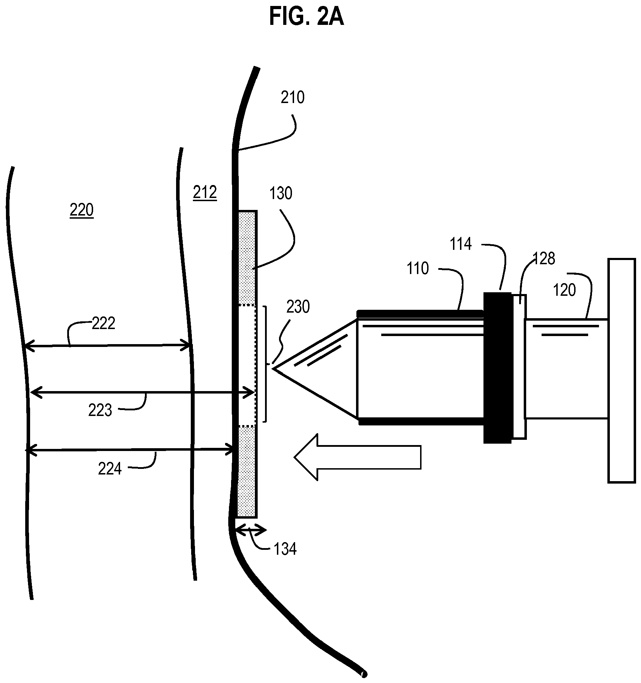

FIG. 1C is a block diagram that illustrates an example system base 130, according to an embodiment. System base 130 has diameter 131 and includes a system base opening 132 of diameter about equal to the outer diameter 117 of cannula 110. As shown in FIG.2A, system base also has thickness 134. The system base 130 has an area outside the system base opening 132 that is sufficient to inhibit the cannula base 114 from passing into the airway of the target subject. The system base 130 also provides an advantage of locating the entry point incision on the throat of the target subject as a center of the opening 132. The system base 130 is made of any suitable rigid or semi-rigid material, including molded plastic, other types of polymers, stainless steel, titanium, or cobalt chrome, or some combination. In various embodiments, the system base has a diameter in a range from about 5 centimeters to about 15 centimeters. Although appearing circular in FIG. 1C, in various other embodiments, the system base 130 has a different shape, such as a rectangle or shape to match the contours of the neck of the target subject.

FIG. 1D is a block diagram that illustrates an example catheter 140, according to an embodiment. Catheter 140 is a long tube 142 of flexible non-toxic and sterile material, such as silicon plastic or other types of polymers with a fitting 144 for attachment to fluid supply, such as an oxygen supply or a medicine supply. A tube with outer diameter of 2 to 3 millimeters was found suitable for delivering sufficient oxygen to a target subject without exposing the subject to the risks associated with a larger opening, including excessive bleeding, infection and loss of life. The inner diameter is selected in a range from about 1 millimeter to about 2 millimeters. A distal portion is configured to be inserted into an airway of the subject patient and a distal end open to allow free fluid flow, for example into a sub-segmented bronchus of the target subject. A proximal end is configured for attachment to a fluid supply such as an oxygen supply or medicine supply. A proximal portion connects an entry point into the airway of the target subject to the proximal end and also comprises a second inner and outer diameter that are the same as in the distal portion in some embodiments, and different in other embodiments. The second longitudinal inner passage is in fluid communication with the first longitudinal inner passage.

In other embodiments, the kit includes additional or fewer components. For example, in some embodiments, the cannula base 114 has an outer diameter sufficient to prevent falling into any opening for the distal portion 112, and the system base is omitted. In various other embodiments, other components are added, such as those described in more detail below.

FIG. 2A through FIG. 2D are block diagrams that illustrate example use of the components of FIG. 1A through FIG. 1D, according to an embodiment. As shown in FIG. 2A, a cross section of the throat of a target subject is illustrated by skin 210, front wall 212 of airway, and airway 220 having width 222. The system base 130 of thickness 134 is laid on the skin 210 of the subject to expose in opening 132 an entry point 230 for the incision. A distance 224 extends from the distal face of the system base 130 to the back wall of the airway; and a distance 223 extends from the proximal face of the system base 130 to the back wall of the airway.

The trocar 120 has engaged the cannula 110 and straightened the bent distal portion. The proximal face of the cannula base 114 is flush with the distal face of the stop lip 128. The incision is made by driving the trocar engaged with the cannula in the direction of the open arrow.

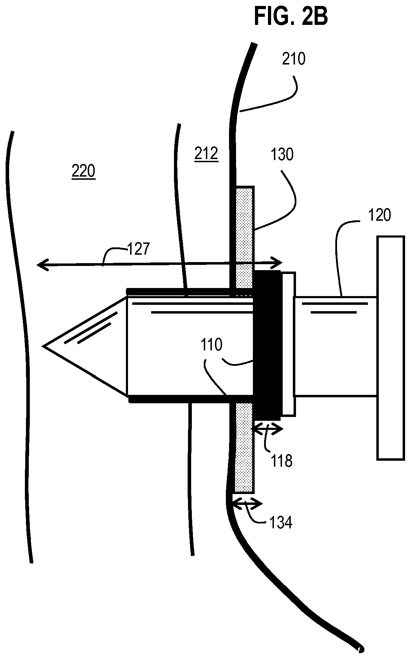

As shown in FIG. 2B, the trocar 120 cuts through the skin 210 and front wall 212 of the target subject airway 220 with the cannula 110 in place. The back wall of the airway is not disturbed so long as the distance from the distal end of trocar to the stop lip 137 minus the thickness 118 of the cannula base minus the thickness 134 of the system base is less than the distance from the skin 210 to the back wall of the airway 220. In some embodiments, markings on cannula base 114 indicate the direction of bending of the cannula with the trocar disengaged. This mark is oriented so that the cannula 110 will bend downward when the trocar is removed.

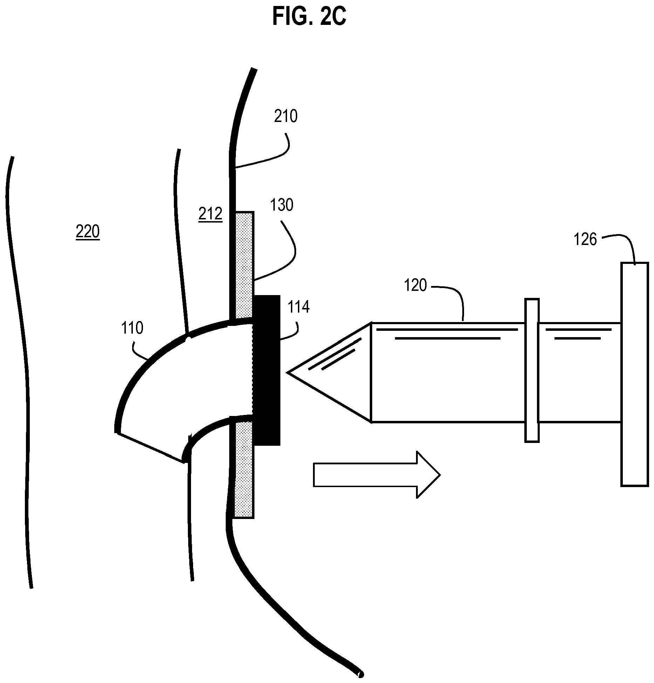

As shown in FIG. 2C, the trocar 120 is removed from the entry site by pulling on handle 126 in the direction of the open arrow. In some embodiments, the base 114 of cannula is held in place while the trocar 120 is removed. The system base 130 prevents the base 114 of cannula 110 from entering the hole made by the cutting edge of the trocar 120. With the trocar disengaged, the distal portion of cannula 110 assumes its original shape, and points downward in the airway towards the lungs of the target subject, as desired.

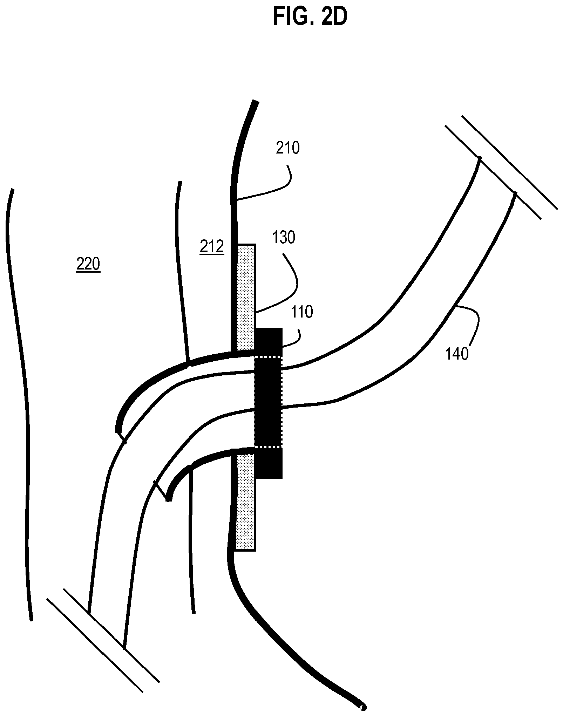

As shown in FIG. 2D, a catheter 140 is inserted through the inner passage of the cannula 110 and is automatically directed downward inside the airway toward the lung because the cannula has remembered its downward bent shape.

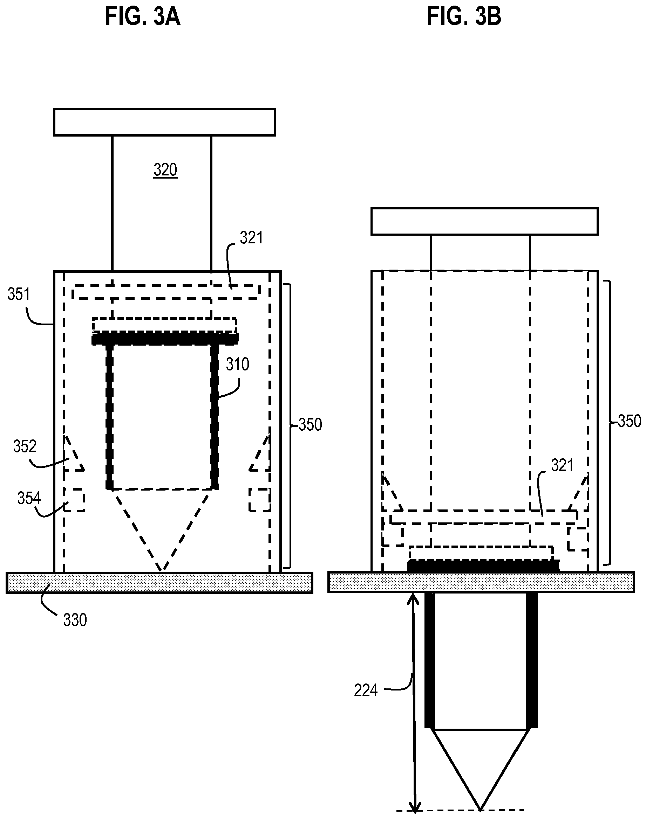

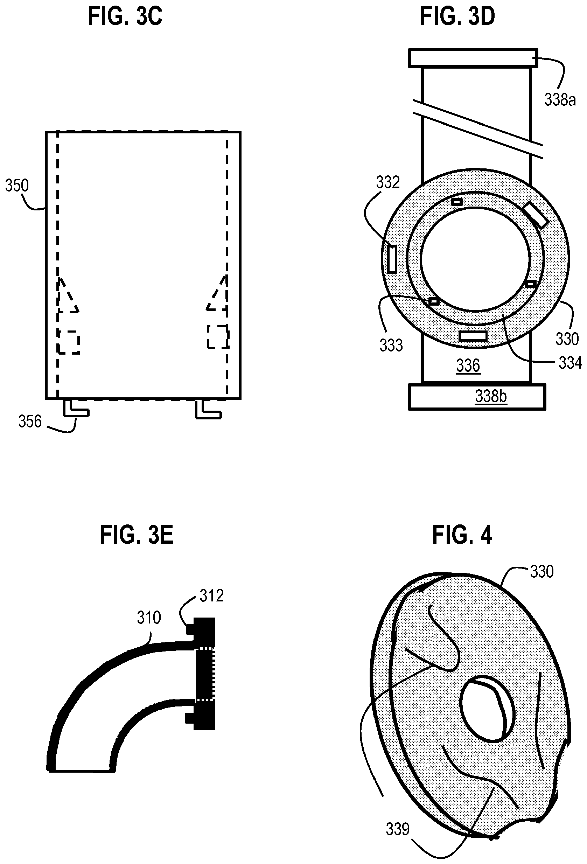

FIG. 3A through FIG. 3E and FIG. 4 are block diagrams that illustrate example variations to the kit of FIG. 1A through FIG. 1D, including a protective casing, according to various embodiments. In some embodiments the device further comprises a protective casing 350. Once the body cavity is penetrated FIG. 3B, the trocar 120 and optional protective casing 140 may be removed. The catheter 110 and base 130 remain in place. In these embodiments, the trocar includes a locking disk 321 that has a diameter larger than stop lip 128. The diameter and length depend on dimensions of any securing mechanism 352 and 354 on the protective casing. The preferred dimensions fix locking disk 321 into the securing mechanism 352 and 354 when in place.

Referring to FIG. 3C, the protective casing 350 comprises an outer casing 351, a securing mechanism 320 for the locking ring of the obturator, and one or more fasteners 352 and 354. Outer casing 351 is made of a generally rigid material, such as metal, plastics or other types of polymers, and has an outer diameter of between about 0.5 centimeters to about 4 centimeters. The interior diameter of outer casing 351 is between about 0.49 centimeters and about 3.99 centimeters. The length of outer casing 351 is between about 0.5 centimeters to about 4 centimeters. Securing mechanism 352 and 354 is located somewhere along the interior surface of the outer casing 351. Securing mechanism 352 and 354 may cover the entire circumference of the outer casing 351 or there may be one or more parts spaced around the circumference of the outer casing 351. The purpose of the securing mechanism is to fix the locking ring of the trocar in place when the trocar tip is inserted into the subject. The securing mechanism also serves as a safety mechanism to prevent the user from pressing the trocar too far into the subject.

In some embodiments the securing mechanism 352 and 354 is located at different locations on the outer casing shaft to conform to different patient sizes. In other embodiments, the locking ring is fixed by the securing mechanism 352 and 354 before the snaps of the cannula are fixed to the base. For these embodiments, the cannula is preferably manually fixed after the trocar and protective casing are removed. It is contemplated that this embodiment will provide for a longer cannula without risking unwanted damage by the trocar.

Referring to FIG. 3C, one or more fasteners 356 are located at the base of the protective casing. The fasteners 356 are used to secure the protective casing 350 to the base as depicted in FIG. 3A and FIG. 3B. In one embodiment, the protective casing 350 is removed from the base by rotating the protective casing 350. In this example the rotation frees the one or more fasteners 356 from the base. Other types of fasteners may be used instead. For example, the fasteners 356 may snap into the base.

FIG. 3D depicts a proximal face of the system base 330. The base 330 comprises a generally disk shaped surface 410. Base 330 further comprises one or more notches 332 for the fasteners of the outer casing. The shape of the notches 332 depends on the configuration of the protective casing fasteners. In one example embodiment the notches 332 are L-shaped to allow for the release of the protective casing when the protective casing is rotated. In some embodiments, the base 330 also comprises of one or more notches 333 which receive one or more snaps 312 of the cannula 310 as depicted in FIG. 3E. The specific configuration of notches 332 and 333 may vary in other embodiments. In consideration of the teaching provided herein, one having ordinary skill in the art would recognize other configurations that while not specifically identified, are still within the overall spirit and scope of this invention.

Referring again to FIG. 3D, in some embodiments the system base 330 is attached to a strap 336 that wraps around the neck of the target subject, and is held in place by complementary buckle 338a and clasp 338b. In some embodiments, strap 336 is attached to system base 330 by eyelets that form part of base 330. In the illustrated embodiment, the system base 330 includes an inner annulus 334 that is slightly recessed for accepting the distal face of cannula base 114.

FIG. 4 depicts a distal face of the system base 330, according to some embodiments. In the illustrated embodiment, the distal face is contoured with one or more contours 339 so that the system base 330 settles most securely when the opening of the system base is properly positioned over a preferred entry point. Thus, a system base is configured with a shape that follows contours of a throat of the target subject so that the system opening is centered on a location appropriate as an entry site for a catheter for emergency apneic oxygenation.

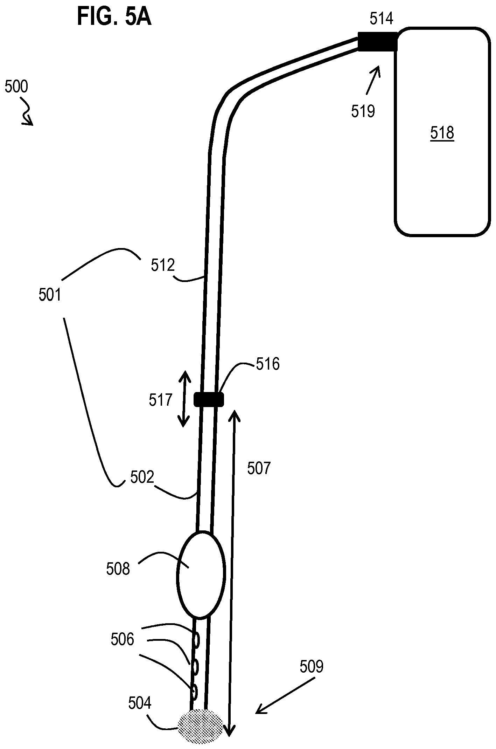

FIG. 5A through FIG. 5H are block diagrams that illustrate example variations in catheters from that depicted in FIG. 1D, according to various embodiments. FIG. 5A is a block diagram that illustrates an apneic oxygenation catheter 500 according to an embodiment. Catheter 500 comprises an elongated shaft 501, having a proximal end 519 and a distal end 509. Catheter 500 further comprises one or more ventilation ports called apertures 506 at the distal portion of the shaft and a connection element 514 at the proximal end. The connection element 514 is depicted connected to a fluid supply tank 518, such as an oxygen supply tank. Apertures 506 may be located in any pattern desirable. For example, in some embodiments a plurality of apertures are located within a particular distance of the distal end of the distal portion, wherein each aperture is configured to permit fluid flow between the first longitudinal inner passage and an outside of the catheter. In some of these embodiments, the particular distance is less than a distance from a sub-segmented bronchus of the target subject to a mainstem bronchus of the target subject.

In some embodiments, the catheter 500 includes a collar 516 to mark the desired length 507 of the catheter to be inserted through the cannula and into the airway of the target subject. The collar is configured to be placed around the catheter at a particular distance 507 to the proximal side from the distal end of the distal portion, wherein the particular distance 507 is approximately equal to a distance from an entry point into the airway of the target subject to a sub-segmented bronchus of the target subject. In some embodiments, the particular distance is in a range from about 5 centimeters to about 15 centimeters. In some embodiments, the collar is moveable along that range. In some embodiments, gradation marks are included along the shaft in addition to or instead of the collar 516. An advantage of the collar 516 is that the collar presents a physical stop when it encounters the cannula. This physical stop allows an operator to detect, without having to look at the catheter, when sufficient length has been inserted into the airway. In some embodiments, the inner and outer diameter of the catheter have one set of values on a distal portion 502 to the distal side of the collar 516, and another set of values on a proximal portion 512 to the proximal side of the collar 516.

Oxygenation catheter 500 advantageously includes padding 504 at the distal end. Padding 504 in various embodiments includes, for example, a balloon, a sponge, or other attachment that would help prevent injury to the trachea or bronchi during insertion or dispense air in 360 degrees or both, in some combination. In some embodiments, a dissolvable capsule at the distal end is used to reduce the risk of injury when the device is inserted, alone or in combination with the padding.

Catheter 500 may further comprise one or more balloon 508 along the shaft 502. The purpose of balloon 508 is to secure the device in the patient, in some embodiments; or to concentrate the oxygen to a certain area of the lungs, in some embodiments. The one or more balloons 508 may be located at various locations along the length of shaft 502 depending on the particular needs. Balloon 508 may be inflated using the oxygen source or it may have a separate lumen in which a separate inflation device is attached.

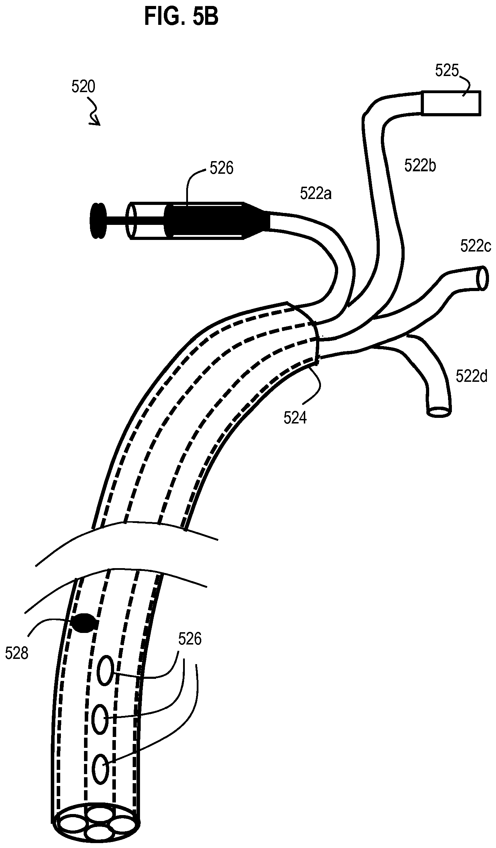

The apneic oxygenation catheter may have more than one lumen. FIG. 5B depicts a quadruple lumen embodiment 520 having a drug delivery lumen 522a and an oxygen delivery lumen 522b inside catheter sheath 524 with apertures 526 into the lumen 522b. In other embodiments, the catheter may have one, two, three or more lumens. The proximal end of drug delivery lumen 522a is configured to be readily attached to containers 526 of drugs. Catheter 520 in some embodiments further comprises a fenestrated diaphragm 528 inside drug delivery lumen 522a. Diaphragm 528 enables small drug particle dispersion for better lung absorption. Example drugs that may be used include, but are not limited to, epinephrine, atropine, and lidocaine. Thus, each of the first longitudinal inner passage in a distal portion and the second longitudinal inner passage in a proximal portion is divided into a plurality of lumens, each lumen in the first longitudinal passage in fluid communication with a corresponding lumen in the second longitudinal passage.

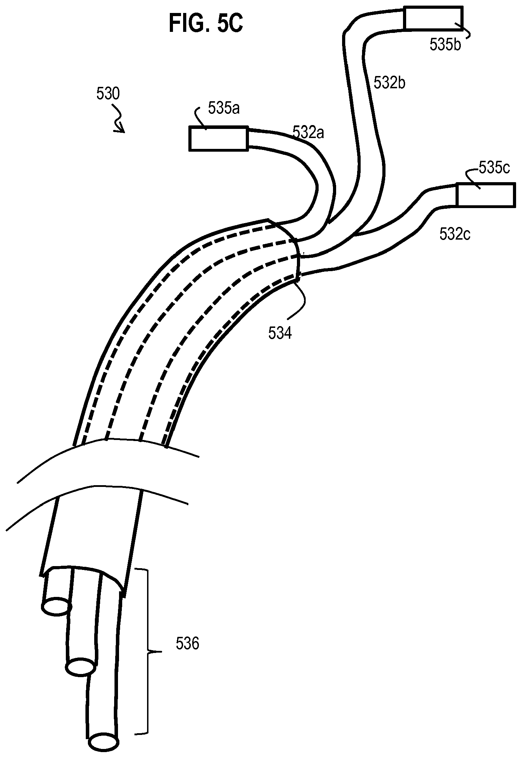

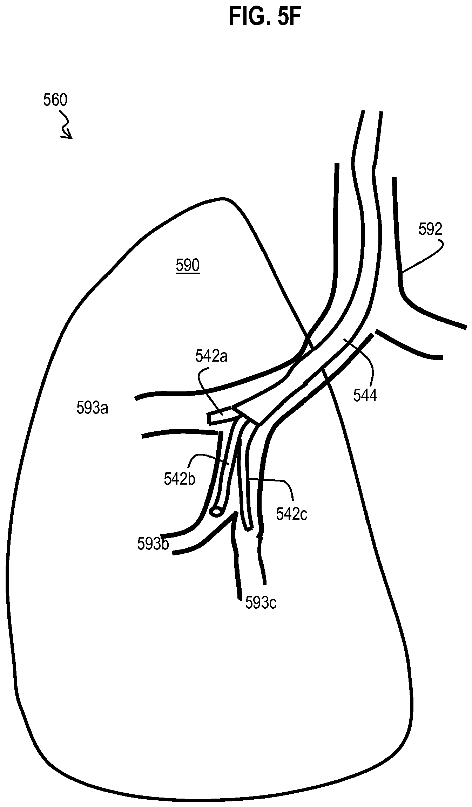

The oxygenation catheter may also have a bifurcated or trifurcated distal end, below a catheter sheath, to provide for additional oxygenation. FIG. 5C is a block diagram that illustrates an example trifurcated catheter 530 with three lumens 532a, 532b and 532c, each configured with connectors 535a, 535b, 535c, respectively, for connecting to a fluid supply tanks, such as an oxygen tank. FIG. 5F is a block diagram that illustrates an example trifurcated catheter 544 deployed in a lung 590. The branch may be located at the base of the trachea or further down in the lung 590. FIG. 5F depicts a branch in one mainstem bronchus, with each different lumen 542a, 542b, 542c, located in a different sub-segmented bronchus of bronchi 593a, 593b, 593c, respectively.

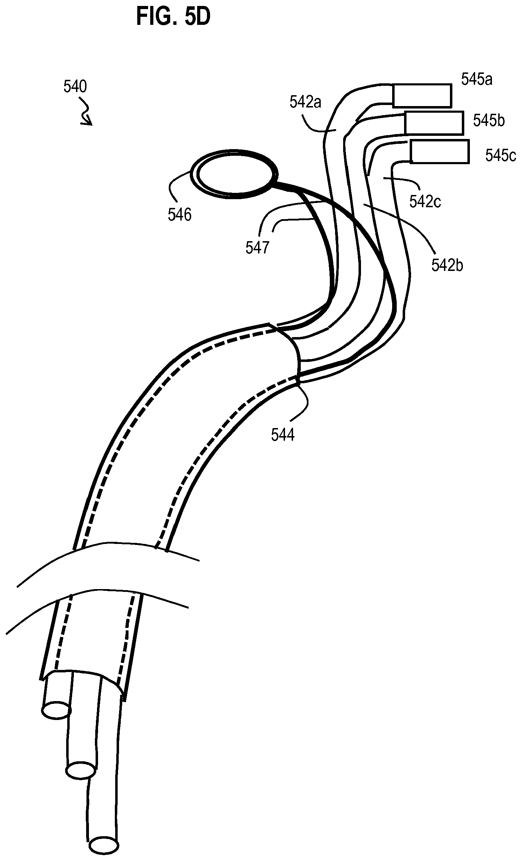

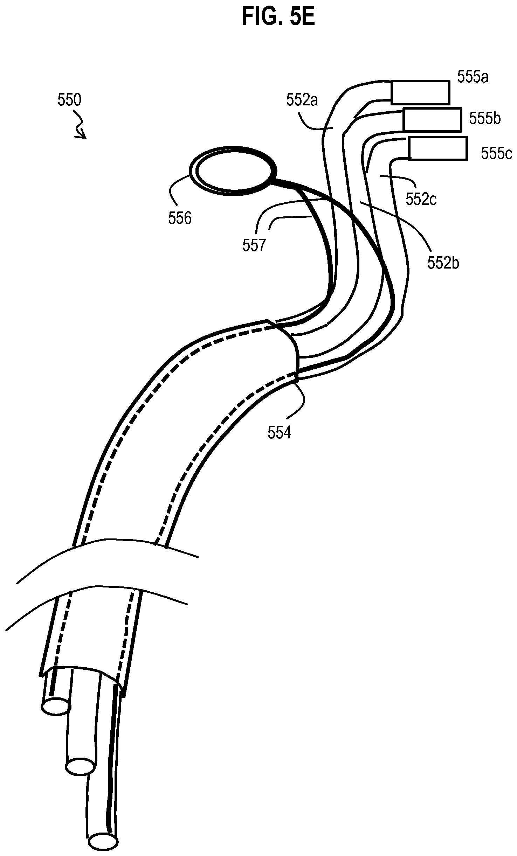

Referring now to FIG. 5D and FIG. 5F, one possible deployment method for the trifurcated system is a pull string. In FIG. 5D separate lumen 542a, 542b, 542c with connectors 545a, 545b, 545c, respectively, are controlled at the distal end by pull string ring 546 connected to pull strings 547. When pulled, the strings 547 retract a sheath 544 of the catheter, exposing each lumen in succession. By feel, the operator may leave one lumen near each of different sub-segmented bronchi. In FIG. 5E, separate lumen 552a, 552b, 552c with connectors 555a, 555b, 555c, respectively, within sheath 554, are controlled at the distal end by pull string ring 556 connected to pull strings 557. When pulled, the strings 557 retract each lumen in succession. Here, each of one or more distal ends (branches) is connected to a pull string 557 to coordinate the deployment of each distal end of lumen 552a, 552b, 552c.

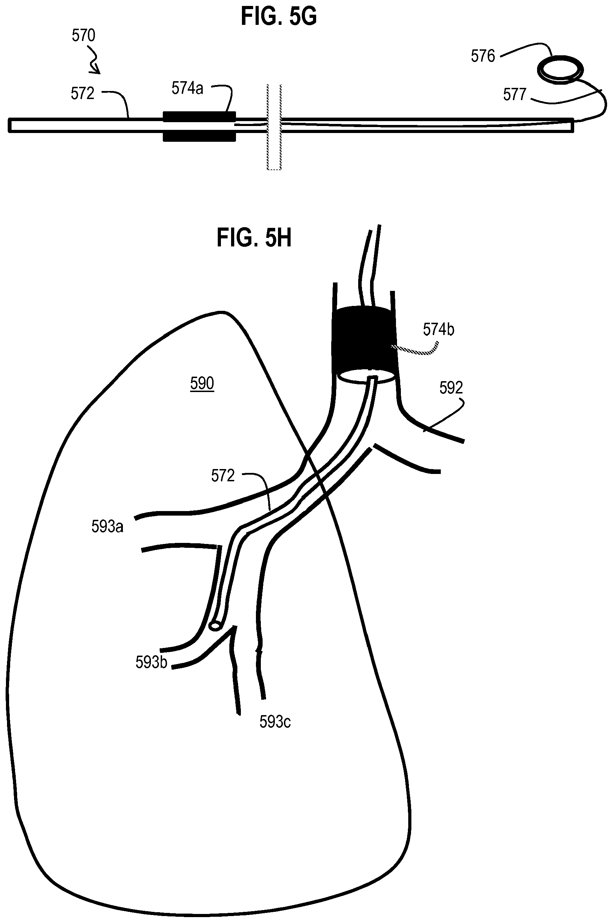

FIG. 5G is a block diagram that illustrates an example alternative securing mechanism to the balloon 508 of FIG. 5A. Catheter 570 includes a shaft 572 and expandable device 574a connected by guide wire 577 to ring 576. When ring 576 is pulled, expandable device 574a expands. Expandable device 574a is used to anchor the catheter 570 in the airway of the target subject, as depicted in FIG. 5H. FIG. 5H is a block diagram that illustrates an example location of catheter shaft 572 and expanding device 574b in an expanded configuration in a lung 590 of a target subject. The distal end of shaft 572 is located in sub-segmented bronchus 593b of sub-segmented bronchi 593a, 593b, 593c. In an example embodiment, expandable device 574a is made of nitinol. However, other biocompatible materials, such as metals, plastics or other polymers, or some combination, are used in other embodiments. In some embodiments, expandable device 574a is a silicon balloon or similar type feature is used. Catheter 570 further comprises a guide wire channel in some embodiments.

Thus various embodiments include an anchoring device disposed outside the catheter at a particular distance proximal to the distal end of the distal portion of the catheter, wherein the anchoring device is configured to assume a first shape of small cross sectional area and a second shape of larger cross sectional area sufficient to fill the airway of the target subject outside the catheter.

Various combinations of the devices described above may be combined into a kit for emergency use. In addition to the oxygenation catheter and a cannula-trocar crycothyrotomy intubation assembly, a kit may further comprise an oxygen source. It is contemplated that an oxygen tank capable of containing enough oxygen to maintain an average sized patient for at least an hour would be preferable. However, larger or smaller tanks may be used in the kit. A person having ordinary skill in the art would be capable of determining the most appropriate tank size. In some example embodiments, vials of drugs such as, for instance, epinephrine, atropine, or lidocaine are provided with the kit.

A method is described for providing apneic oxygenation, according to some examples. Although steps are described as integral steps in a particular order for purposes of illustration, in other examples, one or more steps, or portions thereof, are performed in a different order, or overlapping in time, in series or in parallel, or are omitted, or one or more additional steps are added, or the method is changed in some combination of ways. A method for emergency apneic oxygenation includes cutting an opening of diameter less than 10 millimeters into an airway of a target subject at an entry site. The method also includes passing a distal end of a catheter through the opening and down the airway of the target subject to a sub-segmented bronchus of the target subject. The method still further includes connecting a distal end of the catheter to a supply of oxygen, and providing oxygen from the supply to the target subject at a rate sufficient to sustain life of the target subject.

In some examples, cutting the opening further comprises inserting at the entry site a trocar engaged with a cannula comprising a distal end of shape memory material, wherein the cannula without trocar engaged is bent in a first direction. The trocar is inserted so that the first direction is directed downward in the airway of the target subject. The step further includes removing the trocar while leaving the cannula inserted at the entry site.

In some examples, inserting the trocar engaged with the cannula at the entry site further includes placing a system base on a throat of the target subject so that an opening of the system base is centered on the entry site, and inserting the trocar engaged with the cannula through the opening in the system base.

In some examples, passing the distal end of the catheter through the opening further comprises passing the distal end of the catheter through the cannula.

In some examples, the opening into the airway of the target subject is in a range from about 2 millimeters to about 3 millimeters.

In the foregoing specification, the invention has been described with reference to specific embodiments thereof. It will, however, be evident that various modifications and changes may be made thereto without departing from the broader scope of the invention as defined by the appended claims. The specification and drawings are, accordingly, to be regarded in an illustrative rather than a restrictive sense. Throughout this specification and the claims, unless the context requires otherwise, the word "comprise" and its variations, such as "comprises" and "comprising," will be understood to imply the inclusion of a stated item, element or step or group of items, elements or steps but not the exclusion of any other item, element or step or group of items, elements or steps. Furthermore, the indefinite article "a" or "an" is meant to indicate one or more of the items, elements or steps modified by the article.

## Claims

1. A cannula (110) for emergency apneic oxygenation comprising: a longitudinal inner passage having an inner diameter;
a distal portion having a first outer diameter (117) greater than the inner diameter (116), wherein the cannula is shaped in the distal portion (112) to bend in a first direction along the inner passage, and wherein the distal portion is made of shape memory material; and a cannula base (114),
wherein the cannula base comprises a distal face having a second outer diameter greater than the first outer diameter, and wherein
a distance (119) from a distal end of the cannula to a proximal end of the distal portion of the cannula is less than a distance from a surface of a throat of a target subject to a distal surface of an airway of the target subject, and
the inner passage is configured to pass a catheter connected at a proximal end to an oxygen source.

2. The system as recited in claim 1, further comprising a straight portion (115) between the cannula base and the distal portion, wherein a diameter of the straight portion is about equal to the first outer diameter.

3. A system for emergency apneic oxygenation comprising:
a cannula according to claims 1 or 2 and a trocar
wherein
the trocar (120) is configured to engage the cannula by passing through the inner passage and straightening the bent distal portion of the cannula,
when the trocar is engaged, a distance from a distal end of the distal portion of the trocar to a proximal end of the distal portion of the cannula is less than a distance from a surface of a throat of a target subject to a distal surface of an airway of the target subject.

4. A system as recited in claim 3, further comprising a system base (130) that has a system base opening (132) that has a diameter (117) about equal to the first outer diameter, wherein the system base has an area outside the system base opening that is sufficient to inhibit the cannula base from passing into the airway of the target subject.

5. A system as recited in claim 4, wherein the system base is configured with a shape that follows contours of a throat of the target subject so that the system opening is centered on a location appropriate as an entry site for a catheter for emergency apneic oxygenation.

6. A system as recited in any of claims 3 - 5, further comprising a catheter (140) configured to pass through the inner passage of the cannula and be directed by the direction of the bent distal portion of the cannula down the airway of the target subject after the cannula passes into the airway of the target subject and the trocar is removed.

7. The system as recited in claims 4-6, wherein the distal face of the cannula base is configured to attach to a proximal face of the system base outside the opening in the system base.

8. A kit for emergency apneic oxygenation comprising:
a cannula according to claims 1 or 2,
a trocar configured to engage the cannula by passing through the inner passage; and
a system base (130) having an opening (132) about equal to the first outer diameter and configured to be placed with the opening centered on an appropriate entry site on a target subject for the trocar engaged with the cannula; and
a catheter (140) configured to pass through the cannula after insertion of the cannula into the entry site by the engaged trocar and subsequent removal of the trocar, wherein the catheter has a length that is at least a sum of a first distance from the entry site to a sub-segmented bronchus of the target subject and a second distance from the entry site to a supply of fluid.

9. A cannula, system or kit as recited in any preceding claim, wherein the first outer diameter is less than 10 millimeters.

10. A cannula, system or kit as recited in any preceding claim wherein the inner diameter is in a range from about 2 to about 3 millimeters.

11. A kit as recited in any of claims 8-10, wherein, when the trocar engaged with the cannula is disposed through the system base, a distance from the distal face of the system base to a distal end of the trocar is less than a distance from a surface of a throat of the target subject to a distal surface of an airway of the target subject.

12. A kit as recited in any of claims 8 -11, wherein the fluid is oxygen.

13. A kit as recited in any of claims 8 - 12, wherein the catheter is divided into a plurality of lumen.

14. A kit as recited in any of claims 8 -13, wherein the catheter is one of a plurality of catheters (552)_encompassed by a sheath (524), and the inner diameter of the cannula is not less than a diameter of the sheath.

15. A kit as recited in claim 14, wherein corresponding distal ends of the plurality of catheters are configured to be separated to each enter a corresponding sub-segmented bronchus when the cannula is deployed in the airway of the target subject and the sheath is configured through the inner passage of the cannula.

## Patentansprüche

1. Kanüle (110) zur Notfall-Apnoe-Sauerstoffzufuhr, die Folgendes umfasst: einen längs verlaufenden inneren Durchgang, der einen Innendurchmesser aufweist,
einen distalen Abschnitt, der einen ersten Außendurchmesser (117) aufweist, der größer ist als der Innendurchmesser (116), wobei die Kanüle in dem distalen Abschnitt (112) so geformt ist, dass sie sich in einer ersten Richtung entlang des inneren Durchgangs biegt, und wobei der distale Abschnitt aus Formgedächtnis-Material hergestellt ist, und eine Kanülenbasis (114),
wobei die Kanülenbasis eine distale Fläche umfasst, die einen zweiten Außendurchmesser aufweist, der größer ist als der Innendurchmesser, und wobei
eine Entfernung (119) von einem distalen Ende der Kanüle zu einem proximalen Ende des distalen Abschnitts der Kanüle geringer ist als eine Entfernung von einer Oberfläche eines Rachens eines Zielsubjekts zu einer distalen Oberfläche eines Atemweges des Zielsubjekts und
der innere Durchgang dafür konfiguriert ist, einen Katheter, der an einem proximalen Ende mit einer Sauerstoffquelle verbunden ist, zu führen.

2. System nach Anspruch 1,
das ferner einen geraden Abschnitt (115) zwischen der Kanülenbasis und dem distalen Abschnitt umfasst, wobei ein Durchmesser des geraden Abschnitts etwa gleich dem ersten Außendurchmesser ist.

3. System zur Notfall-Apnoe-Sauerstoffzufuhr, das Folgendes umfasst:
eine Kanüle nach Anspruch 1 oder 2 und einen Trokar,
wobei der Trokar (120) dafür konfiguriert ist, durch Hindurchgehen durch den inneren Durchgang und Begradigen des gebogenen distalen Abschnitts der Kanüle die Kanüle in Eingriff zu nehmen,
wenn der Trokar in Eingriff gebracht ist, eine Entfernung von einem distalen Ende des distalen Abschnitts des Trokars zu einem proximalen Ende des distalen Abschnitts der Kanüle geringer ist als eine Entfernung von einer Oberfläche eines Rachens eines Zielsubjekts zu einer distalen Oberfläche eines Atemweges des Zielsubjekts.

4. System nach Anspruch 3, das ferner eine Systembasis (130) umfasst, die eine Systembasisöffnung (132) aufweist, die einen Durchmesser (117) aufweist, der etwa gleich dem ersten Außendurchmesser ist, wobei die Systembasis eine Fläche außerhalb der Systembasisöffnung aufweist, die ausreichend ist, um die Kanüle daran zu hindern, in den Atemweg des Zielsubjekts hindurchzugehen.

5. System nach Anspruch 4, wobei die Systembasis mit einer Form konfiguriert ist, die den Umrissen eines Rachens des Zielsubjekts folgt, so dass die Systemöffnung an einer Position zentriert wird, die als eine Eintrittsstelle für einen Katheter zur Notfall-Apnoe-Sauerstoffzufuhr geeignet ist.

6. System nach einem der Ansprüche 3 bis 5, das ferner einen Katheter (140) umfasst, der dafür konfiguriert ist, durch den inneren Durchgang der Kanüle hindurchzugehen und durch die Richtung des gebogenen distalen Abschnitts der Kanüle den Atemweg des Zielsubjekts hinab geleitet zu werden, nachdem die Kanüle in den Atemweg des Zielsubjekts hindurchgeht und der Trokar entfernt ist.

7. System nach einem der Ansprüche 4 bis 6, wobei die distale Fläche der Kanülenbasis dafür konfiguriert ist, an einer proximalen Fläche der Systembasis außerhalb der Öffnung in der Systembasis befestigt zu werden.

8. Besteck für Notfall-Apnoe-Sauerstoffzufuhr, das Folgendes umfasst:
eine Kanüle nach Anspruch 1 oder 2,
einen Trokar, der dafür konfiguriert ist, durch Hindurchgehen durch den inneren Durchgang die Kanüle in Eingriff zu nehmen, und
eine Systembasis (130), die eine Öffnung (132), etwa gleich dem ersten Außendurchmesser aufweist und dafür konfiguriert ist, so platziert zu werden, dass die Öffnung an einer geeigneten Eintrittsstelle an einem Zielsubjekt für den mit der Kanüle in Eingriff gebrachten Trokar zentriert ist, und
einen Katheter (140), der dafür konfiguriert ist, nach dem Einsetzen der Kanüle in die Eintrittsstelle durch den in Eingriff genommenen Trokar und dem anschließenden Entfernen des Trokars durch die Kanüle hindurchzugehen, wobei der Katheter eine Länge aufweist, die wenigstens eine Summe einer ersten Entfernung von der Eintrittsstelle zu einem unterteilten Bronchus des Zielsubjekts und einer zweiten Entfernung von der Eintrittsstelle zu einer Fluidzufuhr ist.

9. Kanüle, System oder Besteck nach einem der vorhergehenden Ansprüche, wobei der erste Außendurchmesser geringer als 10 mm ist.

10. Kanüle, System oder Besteck nach einem der vorhergehenden Ansprüche, wobei der Innendurchmesser in einem Bereich von etwa 2 bis etwa 3 mm liegt.

11. Besteck nach einem der Ansprüche 8 bis 10, wobei, wenn der mit der Kanüle in Eingriff gebrachte Trokar durch die Systembasis angeordnet ist, eine Entfernung von der distalen Fläche der Systembasis zu einem distalen Ende des Trokars geringer ist als eine Entfernung von einer Oberfläche eines Rachens eines Zielsubjekts zu einer distalen Oberfläche eines Atemweges des Zielsubjekts.

12. Besteck nach einem der Ansprüche 8 bis 11, wobei das Fluid Sauerstoff ist.

13. Besteck nach einem der Ansprüche 8 bis 12, wobei der Katheter in mehrere Lumina geteilt ist.

14. Besteck nach einem der Ansprüche 8 bis 13, wobei der Katheter einer von mehreren Kathetern (552) ist, die von einer Hülle (524) umgeben sind, und der Innendurchmesser der Kanüle nicht geringer ist als der Durchmesser der Hülle.

15. Besteck nach Anspruch 14, wobei entsprechende distale Enden der mehreren Katheter dafür konfiguriert sind, getrennt zu werden, um jeweils in einen entsprechenden unterteilten Bronchus einzutreten, wenn die Kanüle in dem Atemweg des Zielsubjekts eingesetzt wird, und die Hülle durch den inneren Durchgang der Kanüle konfiguriert ist.

## Revendications

1. Canule (110) pour oxygénation apnéique d'urgence, comprenant: un passage longitudinal interne ayant un diamètre intérieur ;
une partie distale ayant un premier diamètre extérieur (117) supérieur au diamètre intérieur (116), dans laquelle la canule est formée dans la partie distale (112) de sorte à se plier dans une première direction le long du passage interne, et dans laquelle la partie distale est composée d'un matériau à mémoire de forme ; et une base de canule (114) ;
dans laquelle la base de la canule comprend une face distale ayant un deuxième diamètre extérieur supérieur au premier diamètre extérieur ; et dans laquelle :
une distance (119) entre une extrémité distale de la canule et une extrémité proximale de la partie distale de la canule est inférieure à une distance entre une surface d'une gorge d'un sujet cible et une surface distale d'une voie respiratoire du sujet cible ; et
le passage interne est configuré pour faire passer un cathéter connecté au niveau d'une extrémité proximale à une source d'oxygène.

2. Système selon la revendication 1,
comprenant en outre une partie droite (115) entre la base de la canule et la partie distale, un diamètre de la partie droite étant à peu près égal au premier diamètre extérieur.

3. Système pour une oxygénation apnéique d'urgence, comprenant :
une canule selon les revendications 1 ou 2 et un trocart ;
dans lequel le trocart (120) est configuré pour s'engager dans la canule en passant à travers le passage interne et en redressant la partie distale pliée de la canule ;
lorsque le trocart est engagé, une distance entre une extrémité distale de la partie distale du trocart et une extrémité proximale de la canule est inférieure à une distance entre une surface d'une gorge du sujet cible et une surface distale d'une voie respiratoire du sujet cible.

4. Système selon la revendication 3, comprenant en outre une base de système (130) comportant une ouverture de la base de système (132) ayant un diamètre (117) à peu près égal au premier diamètre extérieur, la base du système comportant une zone située à l'extérieur de l'ouverture de la base du système, suffisante pour empêcher le passage de la base de la canule dans la voie respiratoire du sujet cible.

5. Système selon la revendication 4, dans lequel la base du système est configurée avec une forme suivant le contour d'une gorge du sujet cible, de sorte que l'ouverture du système est centrée en un emplacement approprié comme site d'entrée d'un cathéter pour oxygénation apnéique d'urgence.

6. Système selon l'une quelconque des revendications 3 à 5, comprenant en outre un cathéter (140) configuré pour passer à travers le passage interne de la canule et être dirigé par la direction de la partie distale pliée de la canule, vers le bas le long de la voie respiratoire du sujet cible après le passage de la canule dans la voie respiratoire du sujet cible et le retrait du trocart.

7. Système selon les revendications 4 à 6, dans lequel la face distale de la base de la canule est configurée pour être fixée sur une face proximale de la base du système à l'extérieur de l'ouverture dans la base du système.

8. Kit pour oxygénation apnéique d'urgence, comprenant :
une canule selon les revendications 1 ou 2 ;
un trocart configuré pour s'engager dans la canule en passant à travers le passage interne ; et
une base du système (130) comportant une ouverture (132) à peu près égale au premier diamètre extérieur et configurée pour être disposée de sorte que l'ouverture est centrée sur un site d'entrée approprié sur un sujet cible pour le trocart engagé dans la canule ; et
un cathéter (140) configuré pour passer à travers la canule après l'insertion de la canule dans le site d'entrée par le trocart engagé et le retrait subséquent du trocart, dans lequel le cathéter a une longueur correspondant au moins à une somme d'une première distance entre le site d'entrée et une bronche sous-segmentée du sujet cible et d'une deuxième distance entre le site d'entrée et une alimentation en fluide.

9. Canule, système ou kit selon l'une quelconque des revendications précédentes, dans lequel le premier diamètre extérieur est inférieur à 10 millimètres.

10. Canule, système ou kit selon l'une quelconque des revendications précédentes, dans lequel le diamètre intérieur est compris dans un intervalle allant d'environ 2 à environ 3 millimètres.

11. Kit selon l'une quelconque des revendications 8 à 10, dans lequel, lorsque le trocart en prise avec la canule est disposé à travers la base du système, une distance entre la face distale de la base du système et une extrémité distale du trocart est inférieure à une distance entre une surface d'une gorge du sujet cible et une surface distale d'une voie respiratoire du sujet cible.

12. Kit selon l'une quelconque des revendications 8 à 11, dans lequel le fluide est de l'oxygène.

13. Kit selon l'une quelconque des revendications 8 à 12, dans lequel le cathéter est divisé en plusieurs lumières.

14. Kit selon l'une quelconque des revendications 8 à 13, dans lequel le cathéter est l'un de plusieurs cathéters (552) entourés par une gaine (524), le diamètre intérieur de la canule n'étant pas inférieur à un diamètre de la gaine.

15. Kit selon la revendication 14, dans lequel des extrémités distales correspondantes des plusieurs cathéters sont configurées pour être séparées de sorte à entrer chacune dans une bronche sous-segmentée correspondante lorsque la canule est placée dans les voies respiratoires du sujet cible, la gaine étant configurée à travers le passage interne de la canule.
